(19) Europäisches Patentamt
European Patent Office
Office européen des brevets

(11) Publication number : 0 455 356 A1

(12) # EUROPEAN PATENT APPLICATION

(21) Application number : 91303089.6

(22) Date of filing : 09.04.91

(51) Int. Cl.[5] : A61K 31/41, C07D 285/08, C07D 417/04

(30) Priority : 10.04.90 GB 9008123

(43) Date of publication of application : 06.11.91 Bulletin 91/45

(84) Designated Contracting States : AT BE CH DE DK ES FR GB GR IT LI LU NL SE

(71) Applicant : LILLY INDUSTRIES LIMITED
Kingsclere Road
Basingstoke Hants RG21 2XA (GB)

(72) Inventor : Chakrabarti, Jiban Kumar
3 Holly Hedge Road
Frimley, Camberley, Surrey (GB)
Inventor : Smith, Colin William
36 Avebury
Bracknell, Berks. (GB)
Inventor : Williamson, William Robert Nigel
Littlecote, One Pin Lane
Farnham Common, Slough, Berks. (GB)

(74) Representative : Hudson, Christopher Mark et al
European Patent Attorney Erl Wood Manor
Windlesham Surrey GU20 6PH (GB)

(54) **Substituted 5-amino-1,2,4-thiadiazoles with pharmaceutical activity.**

(57) Pharmaceutical compounds of the following formula are described :

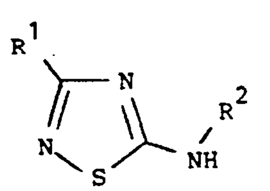

(I)

in which $R^1$ is hydrogen, $C_{1-4}$ alkyl, $C_{1-4}$ alkoxy-$C_{1-4}$ alkyl, hydroxy-$C_{1-4}$ alkyl, optionally substituted phenyl, optionally substituted phenyl-$C_{1-4}$ alkyl or a heterocycle selected from :

EP 0 455 356 A1

Jouve, 18, rue Saint-Denis, 75001 PARIS

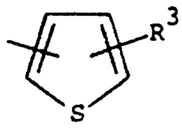
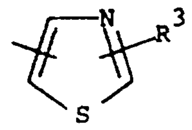
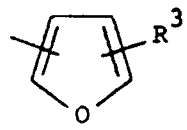
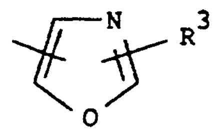
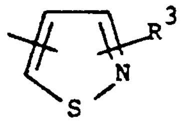
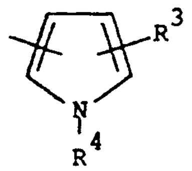
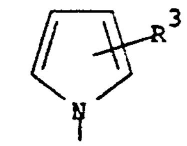
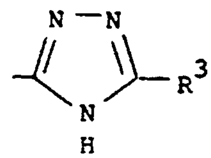
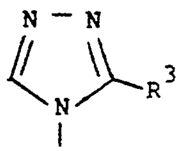
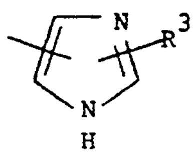
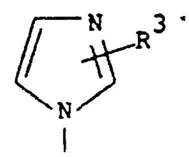
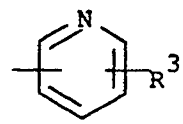
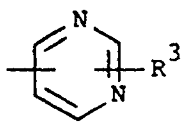
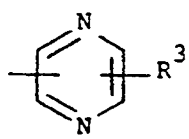
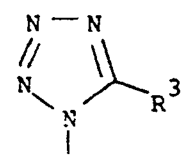
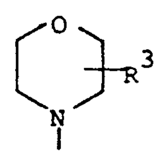
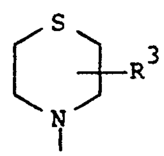

where $R^3$ is hydrogen, $C_{1-4}$ alkyl, $C_{1-4}$ alkoxy, nitro, halo, cyano, trifluoromethyl, carboxyl or $-CONH_2$, and $R^4$ is $C_{1-4}$ alkyl ; and $R^2$ is hydrogen, $C_{1-4}$ alkyl, phenyl or thienyl substituted with a carboxyl or $C_{1-4}$ alkoxy-carbonyl group, an acyl group of the formula $R^5CO$- where $R^5$ is hydrogen, $C_{1-4}$ alkyl, $C_{1-4}$ alkoxy-$C_{1-4}$ alkyl, halo $C_{1-4}$ alkyl, $C_{1-4}$ alkoxy, halo-$C_{1-4}$ alkoxy, phenyl, phenyl-$C_{1-4}$ alkyl, $-NHC_{1-4}$ alkyl, -NH phenyl or

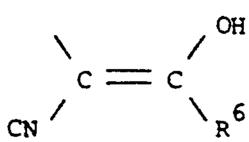

where $R^6$ is $C_{1-4}$ alkyl, or a group of the formula $-CH{=}C(COC_{1-4}\ alkoxy)_2$ ; or a salt thereof.

This invention relates to 1,2,4-thiadiazole compounds and their use as pharmaceuticals.

Certain 1,2,4-thiadiazole compounds are described in the literature. For example, J. Goerdeler and his co-workers disclose 5-amino-3-methyl-1,2,4-thiadiazole, 5-acetamido-3-ethyl-1,2,4-thiadiazole and 5-amino-3-phenyl-1,2,4-thiadiazole in their paper in Chemische Berichte, 1954, 87, 57-68. No biological activity is ascribed to the compounds.

In British Patent 1 257 346 certain 5-amino-3-pyridyl-1,2,4-thiadiazoles are described as intermediates in the production of azo dyestuffs.

The thiadiazole compounds of the invention having pharmaceutical properties are of the formula:

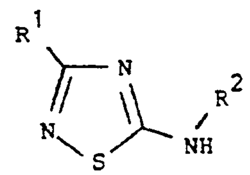

(I)

in which $R^1$ is hydrogen, $C_{1-4}$ alkyl, $C_{1-4}$ alkoxy-$C_{1-4}$ alkyl, hydroxy-$C_{1-4}$ alkyl, optionally substituted phenyl, optionally substituted phenyl-$C_{1-4}$ alkyl or a heterocycle selected from:

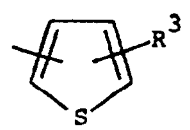 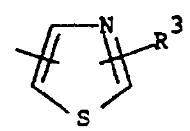 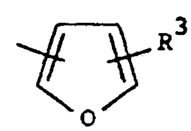 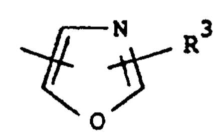

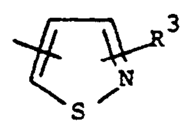 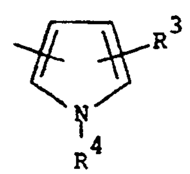 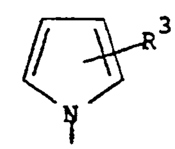 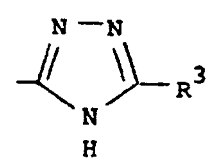

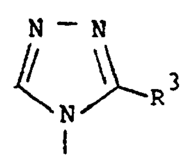 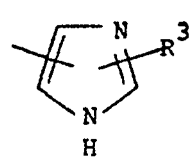 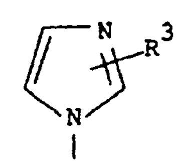 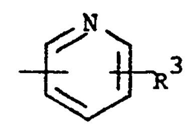

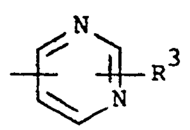 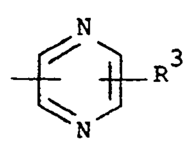 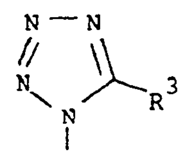 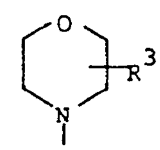

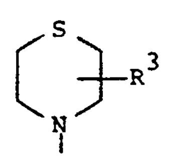

where $R^3$ is hydrogen, $C_{1-4}$ alkyl, $C_{1-4}$ alkoxy, nitro, halo, cyano, trifluoromethyl, carboxyl or $-CONH_2$, and $R^4$ is $C_{1-4}$ alkyl; and $R^2$ is hydrogen, $C_{1-4}$ alkyl, phenyl or thienyl substituted with a carboxyl or $C_{1-4}$ alkoxy-carbonyl group, an acyl group of the formula $R^5CO-$ where $R^5$ is hydrogen, $C_{1-4}$ alkyl, $C_{1-4}$ alkoxy-$C_{1-4}$ alkyl, halo-$C_{1-4}$ alkyl, $C_{1-4}$ alkoxy, halo-$C_{1-4}$ alkoxy, phenyl, phenyl-$C_{1-4}$ alkyl, $-NHC_{1-4}$ alkyl, -NH phenyl or

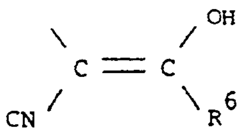

where $R^6$ is $C_{1-4}$ alkyl, or a group of the formula $-CH=C(COC_{1-4}\ alkoxy)_2$; and salts thereof.

The compounds of formula (I) above in which $R^2$ is other than hydrogen are novel, with the exception of compounds in which

(i) $R^1$ is hydrogen and $R^2$ is $CH_3CO$- or phenyl,
(ii) $R^1$ is methyl and $R^2$ is methyl or isopropyl,
(iii) $R^1$ is ethyl and $R^2$ is ethyl, isopropyl or $CH_3CO$-,
(iv) $R^1$ is propyl and $R^2$ is methyl, and
(v) $R^1$ is phenyl and $R^2$ is $C_{1-4}$ alkyl

In the above general formula, reference to $C_{1-4}$ alkyl means any alkyl group containing one to four carbon atoms, for example, methyl, ethyl, propyl, isopropyl, butyl and tertiary butyl, and particularly methyl and ethyl. A halo-$C_{1-4}$ alkyl group is one such alkyl group substituted by one or more halogens preferably chlorine, bromine or fluorine, and examples include $CF_3$- and $CCl_3CH_2$-. A $C_{1-4}$ alkoxy group is of the formula RO- where R is $C_{1-4}$ alkyl as above.

An optionally substituted phenyl group includes unsubstituted phenyl or phenyl substituted with one or more, preferably one to three, substituents chosen from, for example, $C_{1-4}$ alkyl, $C_{1-4}$ alkoxy, nitro, halo, cyano, trifluoromethyl, carboxyl, $C_{1-4}$ alkoxy-carbonyl or $-CONH_2$. The $R^2$ group can be phenyl substituted with a carboxyl or $C_{1-4}$ alkoxy-carbonyl group or thienyl substituted with a carboxyl or $C_{1-4}$ alkoxy-carbonyl group. The thienyl groups is preferably 2-thienyl and both phenyl and thienyl rings may be substituted with additional groups such as those listed above.

Examples of the $R^1$ group when it is a heterocycle include 2-thienyl, 3-thienyl, 2-thiazolyl, 4-thiazolyl, 5-thiazolyl, 2-furyl, 3-furyl, 2-oxazolyl, 4-oxazolyl, 5-oxazolyl, 3-isothiazolyl, 4-isothiazolyl, 5-isothiazolyl, 2-N-alkylpyrrolyl, 3-N-alkylpyrrolyl, 1-pyrrolyl, 2-(1,3,4-triazolyl), 1-(1,3,4-triazolyl), 2-imidazolyl, 4-imidazolyl, 1-imidazolyl, 2-pyridyl, 3-pyridyl, 4-pyridyl, 2-pyrimidyl, 4-pyrimidyl, 5-pyrimidyl, 2-pyrazinyl, 1-tetrazolyl, 4-morpholino, 4-thiamorpholino, or any of these radicals substituted with one or more, preferably a single substituent chosen from the list given above. Preferably the heterocyclic ring is unsubstituted, but when the ring is substituted a preferred substituent is $C_{1-4}$ alkyl, especially methyl or ethyl.

A preferred group of compounds having pharmaceutical activity is one of formula (I) above in which $R^1$ is

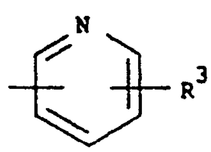

and $R^2$ is hydrogen or an acyl group of the formula $R^5CO$- where $R^5$ is hydrogen or $C_{1-4}$ alkyl.

A further preferred group of compounds having pharmaceutical activity is one of formula (I) above in which $R^1$ is $C_{1-4}$ alkyl and $R^2$ is hydrogen or an acyl group of the formula $R^5CO$- where $R^5$ is hydrogen or $C_{1-4}$ alkyl.

The compounds of the invention are useful both in their free base and acid addition salt forms. The acid addition salts are preferably the pharmaceutically acceptable, non-toxic addition salts with suitable acids, such as those of inorganic acids, for example hydrochloric, hydrobromic, nitric, sulphuric or phosphoric acids, or of organic acids, such as organic carboxylic acids, for example glycollic, maleic, hydroxymaleic, fumaric, malic, tartaric, citric or lactic acid, or organic sulphonic acids for example methane sulphonic, ethane sulphonic, 2-hydroxyethane sulphonic, toluene-p-sulphonic or naphthalene-2-sulphonic acid. In addition to pharmaceutically acceptable acid addition salts, other acid addition salts are included, for example, those with picric or oxalic acid, since they may serve as intermediates in purification or in the preparation of other, for example, pharmaceutically acceptable, acid addition salts, or are useful for identification, characterisation or purification of the free base.

The compounds of formula (I) defined above can be produced by a process which comprises

(a) aminating a compound of the formula

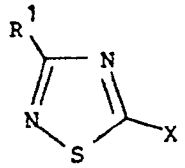

(II)

in which $R^1$ is hydrogen, $C_{1-4}$ alkyl, hydroxy-$C_{1-4}$ alkyl, $C_{1-4}$ alkoxy-$C_{1-4}$ alkyl, optionally substituted phenyl or a heterocycle, and X is a leaving group, with a compound of the formula $R^2NH_2$ where $R^2$ is hydrogen, $C_{1-4}$ alkyl or substituted phenyl or thienyl;

(b) reacting a compound of the formula

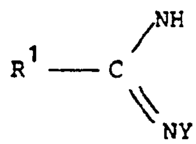

(III)

in which $R^1$ is hydrogen, $C_{1-4}$ alkyl, $C_{1-4}$ alkoxy-$C_{1-4}$ alkyl, optionally substituted phenyl or a heterocycle, and Y is halogen, with a thiocyanate, to give a compound of formula (I) in which $R^2$ is hydrogen; and

(c) acylating a compound of the formula

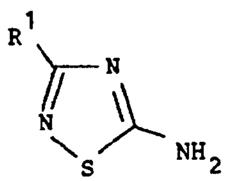

(IV)

in which $R^1$ is hydrogen, $C_{1-4}$ alkyl, $C_{1-4}$ alkoxy-$C_{1-4}$ alkyl, optionally substituted phenyl or a heterocycle, with a reagent of the formula $R^2Z$ where Z is a leaving group, or $(R^2)_2O$, in which $R^2$ is an acyl group of the formula $R^5CO$-.

Compounds of the invention in which $R^2$ is hydrogen or $C_{1-4}$ alkyl can be conveniently prepared by amination of the appropriate intermediate of formula (II) according to process variant (a) above. The reaction is preferably carried out at a temperature of from 10°C to 50°C and in an organic solvent such as tetrahydrofuran.

The intermediate compounds of formula (II) in which X is preferably halogen for example chloro, can be prepared from the appropriate nitrile of formula $R^1$-CN. The nitrile is reacted with sodium methoxide in methanol followed by treatment with acetic acid and ammonium chloride to give an amidino compound which can be cyclised with trihalosulphenyl halide and aqueous sodium hydroxide to give the desired thiadiazole, as follows:

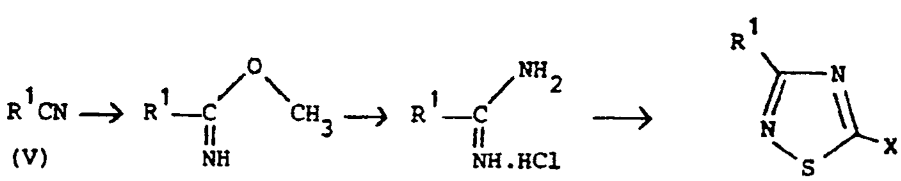

(V)

The nitrile compounds of formula (V) are either known compounds or can be readily prepared by methods known in the art. For example, when $R^1$ is heterocyclic, the nitrile can be prepared from the appropriate halo derivative by formation of a quaternary ammonium salt and reaction with alkali metal cyanide under the influence of heat.

In the process variant (b) above, the intermediate (III) is reacted with thiocyanate, for example, alkali metal or ammonium thiocyanate, with concomitant cyclisation. Preferably the reaction is carried out at a temperature of from -5°C to 10°C and in an organic solvent such as for example methanol.

The intermediate of formula (III) is prepared by reaction of an amidine of formula

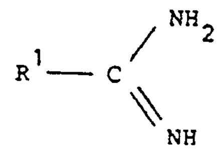

or a salt thereof, with alkali metal alkoxide in an alcohol, for example sodium methoxide in methanol, followed by the action of halogen, preferably bromine. It is usually not necessary or practicable to isolate the halo intermediate of formula (III) and reaction (b) is preferably carried out by first forming the halo intermediate in situ and then reacting it with thiocyanate.

With regard to process variant (c), this process is carried out under conditions suitable for acylation of the free amino group of intermediate (IV), the latter being prepared by process variants (a) or (b). The reaction is preferably performed at a temperature of from 20°C to 90°C and in the presence of an organic solvent such as for example triethylamine The acylating reagent can be an acid anhydride $(R^2)_2O$ or a reagent of the formula $R^2Z$ where Z is a leaving group such as for example, halo, especially chloro or bromo. When the reaction is carried out with a reagent of formula $R^2Z$, it is desirable to employ a base, for example, triethylamine.

Compounds of formula (I) in which $R^1$ is hydroxy-$C_{1-4}$ alkyl can be prepared from the corresponding compounds in which $R^1$ is $C_{1-4}$ alkoxy-$C_{1-4}$ alkyl by dealkylation using a reagent such as for example boron tribromide.

As mentioned above, the compounds have pharmaceutical activity. For example they are active in the adjuvant arthritis test (B.B. Newbould, Chemotherapy of Arthritis Induced in Rats by Mycobacterial Adjuvant, Br.J. Pharmacol. 21, 127-136 (1963)). The compounds for use in the invention disclosed in the following Examples show a reduction of joint swelling and improvement in joint mobility at a dosage of 33 mg/kg in this test. Compounds are also active in tests to measure activity against lipoidal amine-induced arthritis.

The compounds of the invention have also been shown to modify the immune response by inhibiting concanavalin A-induced T-cell proliferation and graft versus host reaction, a T-cell mediated process.

These properties show that the compounds of the invention are indicated for use in the treatment of autoimmune diseases and in the prevention of graft rejection. They have anti-rheumatic properties and are, in particular, indicated for use in the treatment of rheumatoid arthritis and immune diseases such as systemic lupus.

The compounds may be administered by various routes, for example, by the oral or rectal route, topically or parenterally, for example by injection, being usually employed in the form of a pharmaceutical composition. Such compositions form part of the present invention and are prepared in a manner well known in the pharmaceutical art and normally comprise at least one active compound in association with a pharmaceutically acceptable diluent or carrier. In making the compositions of the present invention, the active ingredient will usually be mixed with a carrier, or diluted by a carrier, and/ or enclosed with a carrier which may, for example, be in the form of a capsule, sachet, paper or other container. Where the carrier serves as a diluent, it may be solid, semi-solid, or liquid material which acts as a vehicle, excipient or medium for the active ingredient. Thus, the composition may be in the form of tablets, lozenges, sachets, cachets, elixirs, suspensions, as a solid or in a liquid medium, ointments containing, for example, up to 10% by weight of the active compound, soft and hard gelatin capsules, suppositories, injection solutions and suspensions and sterile packaged powders.

Some examples of suitable carriers are lactose, dextrose, sucrose, sorbitol, mannitol, starch, gum acacia, calcium phosphate, alginates, tragacanth, gelatin, syrup, methyl cellulose, methyl- and propyl-hydroxybenzoiate, talc, magnesium stearate and mineral oil. The compositions of the invention may, as is well known in the art, be formulated so as to provide quick, sustained or delayed release of the active ingredient after adminstration to the patient.

Where the compositions are formulated in unit dosage form, it is preferred that each unit dosage form contains from 5 mg to 500 mg, more usually 25 to 200 mg, of the active ingredient. The term "unit dosage form" refers to physically discrete units suitable as unit dosage for human subjects and animals, each unit containing a predetermined quantity of active material calculated to produce the desired therapeutic effect, in association with the required pharmaceutical carrier.

The compounds are effective over a wide dosage range and for example dosages per day will normally fall within the range of 0.1 to 100 mg/kg, more usually in the range of from 1 to 50 mg/kg or 5 to 100 mg/kg. However, it will be understood that the amount of the compound actually administered will be determined by a physician, in the light of the relevant circumstances including the condition to be treated, the choice of compound to be administered and the chosen route of administration and therefore the above dosage ranges are not intended to limit the scope of the invention in any way.

The invention is illustrated by the following Examples.

EXAMPLE 1

2-Amidinopyridinium hydrochloride

Sodium metal (0.6 g) was dissolved in methanol (225 ml). The resulting solution was cooled to ambient temperature and 2-cyanopyridine (26 g) was added and the solution was stirred for 24 hours. Acetic acid (1.5 ml) was added, followed by ammonium chloride (13.4 g). The suspension was stirred for 24 hours at ambient temperature. The reaction mixture was filtered off from undissolved ammonium chloride, evaporated in vacuo and triturated with ether. The solid was filtered off, dissolved in methanol (50 ml), refiltered. Ethyl acetate (1000 ml) was added, the crystalline solid filtered off, washed with ethyl acetate and dried, m.p. 152-154°C.

The following compounds were made by a similar method:

3-Amidinopyridinium hydrochloride, m.p. 196°C.
Chloroacetamidine hydrochloride, m.p. 101°C.
2-Amidinopyrimidinium hydrochloride, m.p. 196°C
2-Amidinopyrazinium hydrochloride, m.p. 219°C.
1-Thienylacetamidine hydrochloride, m.p. 98-101°C

EXAMPLE 2

5-Amino-3-(2-pyridinyl)-1,2,4-thiadiazole

2-Amidinopyridinium hydrochloride (23.6 g) was dissolved in methanol (200 ml), and to this stirred solution was added a chilled solution of methanol (100 ml) in which sodium (6.9 g) had been dissolved, whilst bromine (7.7 ml) was also added. The temperature was kept below 5°C throughout additions. A solution of potassium thiocyanate (14.6 g) in methanol (100 ml) was added. The exotherm that resulted was allowed to proceed unchecked. This suspension was stirred at ambient temperature for between 1 to 24 hours.

The entire contents of the flask were evaporated, in vacuo, the residue was triturated with a little water, filtered off and washed with a little water. This solid was dissolved in boiling water (1 L), filtered, evaporated to approximately 500 ml and left to cool. The white crystals were filtered off, washed with water and dried. This material was recrystallised from ethanol (300 ml) and, the pure white crystals filtered off, washed with methanol and dried, m.p. 203-204°C.

An equal or improved yield results if potassium thiocyanate is added prior to the addition of the solution of sodium in methanol.

The following compounds were also made:

5-Amino-3-methoxymethyl-1,2,4-thiadiazole, m.p. 140-140.5°C
5-Amino-3-(2-pyrazinyl)-1,2,4-thiadiazole, m.p. 288-290°C
5-Amino-3-(2-pyrimidinyl)-1,2,4-thiadiazole, m.p. 249-251°C
5-Amino-3-n-propyl-1,2,4-thiadiazole, m.p. 89.5-90.5°C
5-Amino-3-(3-pyridinyl)-1,2,4-thiadiazole, m.p. 250-252°C
5-Amino-3-(4-pyridinyl)-1,2,4-thiadiazole, m.p. 298-299°C
5-Amino-3-(2-thienyl)-1,2,4-thiadiazole, m.p. 196-199°C
5-Amino-3-(1,1-dimethylethyl)-1,2,4-thiadiazole, m.p. 158-162°C
5-Amino-3-methoxy-1,2,4-thiadiazole, m.p. 196-199°C.
5-Amino-3-methyl-1,2,4-thiadiazole, m.p. 198.5-200°C.
5-Amino-1,2,4-thiadiazole, m.p. 119.5-120.5°C
5-Amino-3-phenyl-1,2,4-thiadiazole, m.p. 152-156°C.
5-Amino-3-(4-chlorophenyl)-1,2,4-thiadiazole, m.p. 182-182.5°C.
5-Amino-3-(4-methoxyphenyl)-1,2,4-thiadiazole, m.p. 176-176.5°C.
5-Amino-3-[4-(1,1-dimethylethyl)phenyl]-1,2,4-thiadiazole, m.p. 244-244.5°C.
5-Amino-3-(3,4-dichlorophenyl)-1,2,4-thiadiazole, m.p. 199-201°C.
5-Amino-3-[4-(2-methyl)thiazolyl]-1,2,4-thiadiazole, m.p. 224-224°C.
5-Amino-3-(4-morpholino)-1,2,4-thiadiazole, m.p. 203-203.5°C.
5-Amino-3-phenylmethyl-1,2,4-thiadiazole, m.p. 136.5-137.5°C
5-Amino-3-(2-furyl)-1,2,4-thiadiazole, m.p. 163-164°C
5-Amino-3-[4-(2,6-dichloropyridinyl)]-1,2,4-thiadiazole, m.p. 263-263.5°C.

EXAMPLE 3

5-Chloro-3-phenyl-1,2,4-thiadiazole

Pre-dried benzamidine hydrochloride hydrate (36 g) was stirred in chloroform (200 ml) at ambient temeprature, trichloromethane sulphenyl chloride (22 ml) was then added. The suspension was cooled to -5°C and a solution of sodium hydroxide (36 g) in water (90 ml) was added dropwise in four approximately equal portions, leaving the reaction to stir for a short time between each addition, the temperature was never allowed to exceed 5°C.

On completion of additions the reaction mixture was allowed to stir for 15 minutes at 15°C below 10°C, then at ambient temperature for between 1 to 24 hours. The two phase mixture was filtered off, separated, the organic phase was washed three times with brine, dried with magnesium sulphate, filtered, evaporated. The yellow residue was dissolved in hot 40-60°C petroleum ether. The extract was decanted off and chromatographed on a silica gel column using ethyl acetate in petroleum ether (1:4). the fractions containing product were bulked, evaporated in vacuo, when kept under vacuum the yellow oil crystallised, m.p. 49-51°C.

The following compound was made by a similar method; 5-Chloro-3-methyl-1,2,4-thiadiazole, b.p. 45°/18 mm.

EXAMPLE 4

5-Bromo-2-[3-phenyl-5-(1,2,4-thiadiazolyl)]aminobenzoic acid

5-Chloro-3-phenyl-1,2,4-thiadiazole (5.9 g) and 2-amino-5-bromobenzoic acid (4.32 g) were heated, under reflux, under a nitrogen atmosphere, in 2-methoxyethanol (60 ml) for 54 hours. The solution was evaporated in vacuo, the residue was triturated with water, the solid was filtered off, washed with water, then 40-60° petroleum ether, and dried. The solid was suspended in boiling ethanol, chilled, filtered off, washed with ethanol, dissolved in 50°% sodium hydroxide (12 ml) diluted with water (150 ml), and with heating just enough ethanol was added to obtain a solution which was filtered. Acetic acid was added (10 ml), the solution was chilled. On standing crystals were deposited, these were, filtered off, washed dried, m.p. >260°C (dec.).

EXAMPLE 5

5-Methylamino-3-phenyl-1,2,4-thiadiazole

5-Chloro-3-phenyl-1,2,4-thiadiazole (1.97 g) was dissolved in tetrahydrofuran (70 ml) and to this, with stirring, 30% w/v aqueous methylamine (3.73 ml) was added dropwise. The solution was stirred at ambient temperature for 2 hours, evaporated in vacuo, the residue was suspended in hot water, chilled, the solid was filtered off, washed with water and dried. This solid was recyrstallised from toluene to which hexane was added, m.p. 159-160.5°C.

The following compounds were made by a similar method:

5-Ethylamino-3-phenyl-1,2,4-thiadiazole, m.p. 76.5-77.5°C.

5-Methylamino-3-methyl-1,2,4-thiadiazole, m.p. 197.5-198.5°C.

EXAMPLE 6

2-Cyano-3-hydroxy-N-[3-(2-pyridinyl)-5-(1,2,4-thiadiazolyl)]-butenamide

5-Amino-3-(2-pyridinyl)-1,2,4-thiadiazole (7.13 g) was stirred in tetrahydrofuran (100 ml) and to this suspension was added triethylamine (18 ml), followed by a solution of 5-methyl-4-isoxalolyl chloride (11.64 g) in tetrahydrofuran (25 ml). This solution was heated under reflux for 3 hours. The cooled solution was evaporated in vacuo and the residue triturated with water, filtered off and dried. The black solid was suspended in boiling ethyl acetate, cooled, filtered off, dissolved in hot chloroform and chromatographed using 5% methanol as eluant on silica gel. The bulked fractions were evaporated in vacuo.

This triethylamine salt was dissolved in boiling water (500 ml), chilled, filtered and enough acetic acid was added to neutralise this solution (2.3 ml). The precipitated solid was filtered off, washed with water and dried, m.p. >328°C.

The following compounds were also made:

5-Acetamido-3-methyl-1,2,4-thiadiazole, m.p. 149-149.5°C.

2,2,2-Trichloroethyl, 5-(1,2,4-thiadiazolyl)carbamate, m.p. 169.5-171°C.

5-(2-Methoxy)acetamido-3-phenyl-1,2,4-thiadiazole, m.p. 114.5-115°C.

2,2,2-Trichloroethyl, [3-(4-chlorophenyl)-5-(1,2,4-thiadiazolyl)]-carbamate, m.p. 196-196.5°C.

2,2,2-Trichloroethyl, [3-phenyl-5-(1,2,4-thiadiazolyl)]carbamate, m.p. 166-168°C.

3-Phenyl-5-trifluoracetamido-1,2,4-thiadiazole, m.p. 117-119°C.

3-Methyl-5-phenylacetamido-1,2,4-thiadiazole, m.p. 117-118°C.

EXAMPLE 7

Ethyl, 5-ethyl-2-[3-phenyl-5-(1,2,4-thiadiazolyl)]aminothiophene-3-carboxylate

Sodium hydride (50% dispersion in oil, 2.44 g) was stirred in tetrahydrofuran (30 ml) under nitrogen, cooled in an ice bath and treated with a tetrahydrofuran solution (40 ml) of 5-chloro-3-phenyl-1,2,4-thiadiazole (5 g) and ethyl, 2-amino-5-ethylthiophene-3-carboxylate (5.06 g) at a rate that maintained the solution temperature between 5 and 10°. The mixture was stirred in the ice bath for 3 hours and at room temperature over night. The solution was poured onto ice (400 ml), treated with 2M HCl (15.5 ml) and the resulting solid was filtered off, washed with $H_2O$ (2 x 200 ml) and dried at 40°/20 mm. It was recrystallised twice from light petroleum (b.p. 60-80°) (using decolourising charcoal) to give the above ester, m.p. 113°.

EXAMPLE 8

Diethyl, N-{[(3-methyl-1,2,4-thiadiazol-5-yl)amino]methylene}-malonate

5-Amino-3-methyl-1,2,4-thiadiazole (1.78 g) and diethyl ethoxymethylene malonate (2.38 g), in anisole (50 ml) were refluxed for 4 hours. The ethanol evolved was collected in a Dean and Stark head. The solution was evaporated to dryness and the product was recrystallised from light petroleum (b.p. 80-100°) using charcoal to give the diester, m.p. 97°C.

The following compound was made by a similar method:

Diethyl, N-{[(3-phenyl-1,2,4-thiadiazol-5-yl)amino]methylene}-malonate, m.p. 145°C.

EXAMPLE 9

1-Phenyl-3-[(3-phenyl)-5-(1,2,4-thiadiazolyl)] urea

5-Amino-3-phenyl-1,2,4-thiadiazole (2.3 g) was stirred and heated in xylene (50 ml) to 115°. Phenyl isocyanate (1.55 g) was added in xylene (10 ml). The mixture was stirred and refluxed for 1 hour. It was evaporated to dryness in vacuo and crystallised from EtOH twice to give the title compound, m.p. 302-303°C.

EXAMPLE 10

Alternative methods for the preparation of amidine intermediates

(1) 4-Methoxybenzamidine tetrafluoroborate

4-Methoxybenzamide (20.36 g) suspended in dichloromethane (267 ml) was treated rapidly with triethoxytetrafluoroborate (29.05 g) in dichloromethane (67 ml). The mixture cleared in 15 minutes, it was stirred at ambient temperature for 72 hours, evaporated to 1/3 of original volume and treated with dry diethyl ether (667 ml) to give the 4-methoxy phenyl imino ether fluoroborate as white crystals (33.63 g), m.p. 131-132°C.

This product (21.47 g) was added to ammonia (3.09 g) in ethanol (31 ml, 9.7°% solution) and stirred in a closed vessel at room temperature for 91 hours. The solution was evaporated to dryness in vacuo to give 4-methoxybenzamidine tetrafluoroborate as white crystals, m.p. 161-164°C.

(2) 1,5-Dimethyl-2-pyrroleiminoethylether, hydrochloride:

1,5-Dimethyl-2-pyrrolecarbonitrile (24.03 g) in ethanol (9.99 g) and toluene (25 ml) were cooled in ice and treated with gaseous hydrogen chloride until 8.52 g of HCl had been taken up. The mixture was kept in a stoppered flask for 91 hours. The resulting solid iminoether hydrochloride (11.95 g), m.p. 210° (with sublimation + decomposition) was filtered off, washed with toluene and dried in vacuo at room temperature.

The above product (11 g) was added to a solution of ammonia (1.58g) in ethanol (16.3 ml) and kept in a stoppered flask at room temperature (with stirring for the first 24 hours) for 3 weeks. The mixture was filtered to remove ammonium chloride and the filtrate evaporated to dryness to give the title compound (after drying in vacuo at room temperature) as a solid, m.p. 200°.

The following compounds were made by a similar method.

2-Thienylcarbamidine hydrochloride, m.p. 168°C.

Methoxyacetamidine hydrochloride, m.p. 67°C.

n-Butyramidine hydrochloride, m.p. 96°C.

EXAMPLE 11

The following pharmaceutical formulations are given by way of example

(i) Injection formulation

An injection formulation containing 5 mg/ml of active ingredient is prepared from the following

| | |
|---|---|
| Active ingredient | 250 mg |
| 0.1 M Sodium hydroxide | 10 ml |
| N/10 Hydrochloric acid | 2 ml |
| 5% Poloxamer F68 in isotonic saline to | 50 ml |

(ii) Hard gelatin capsule formulation

| | |
|---|---|
| Active ingredient | 100 mg |
| 1% Silicone starch | 50 mg |
| Starch flowable | 50 mg |

(iii) Tablet formulations

| | |
|---|---|
| Active ingredient | 100 mg |
| Microcrystalline cellulose | 185 mg |
| Carboxymethyl cellulose sodium (crosslinked) | 3 mg |
| Povidone | 10 mg |
| Magnesium stearate | 1 mg |

## Claims

1. A compound of the formula

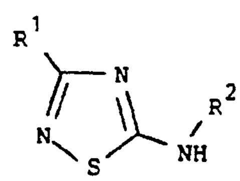

(I)

in which $R^1$ is hydrogen, $C_{1-4}$ alkyl, $C_{1-4}$ alkoxy-$C_{1-4}$ alkyl, hydroxy-$C_{1-4}$ alkyl, optionally substituted phenyl, optionally substituted phenyl-$C_{1-4}$ alkyl or a heterocycle selected from:

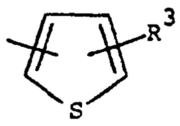 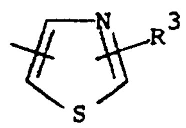 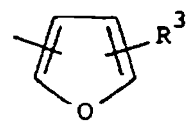 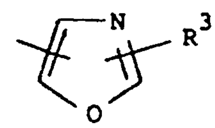

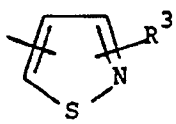 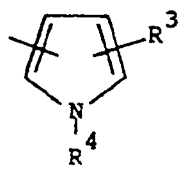 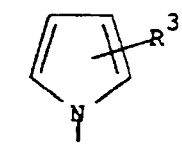 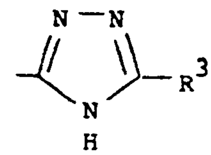

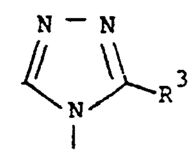 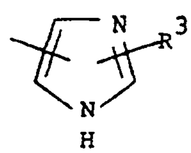 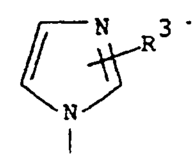 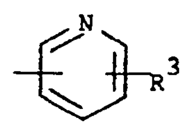

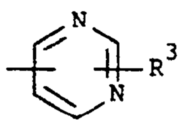 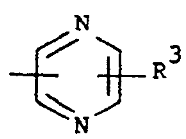 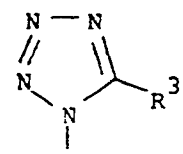 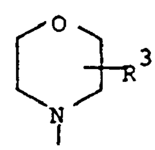

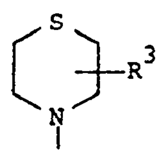

where $R^3$ is hydrogen, $C_{1-4}$ alkyl, $C_{1-4}$ alkoxy, nitro, halo, cyano, trifluoromethyl, carboxyl or $-CONH_2$, and $R^4$ is $C_{1-4}$ alkyl; and $R^2$ is hydrogen, $C_{1-4}$ alkyl, phenyl or thienyl substituted with a carboxyl or $C_{1-4}$ alkoxy-carbonyl group, an acyl group of the formula $R^5CO$- where $R^5$ is hydrogen, $C_{1-4}$ alkyl, $C_{1-4}$ alkoxy-$C_{1-4}$ alkyl, halo $C_{1-4}$ alkyl, $C_{1-4}$ alkoxy, halo-$C_{1-4}$ alkoxy, phenyl, phenyl-$C_{1-4}$ alkyl, $-NHC_{1-4}$ alkyl, -NH phenyl or

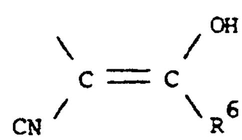

where $R^6$ is $C_{1-4}$ alkyl, or a group of the formula $-CH=C(COC_{1-4}\text{ alkoxy})_2$; or a salt thereof; for use as a pharmaceutical.

2. The use of a compound as defined in claim 1 for the manufacture of a medicament for the treatment of an autoimmune disease or in the prevention of graft rejection.

3. A compound according to claim 1 in which $R^1$ is

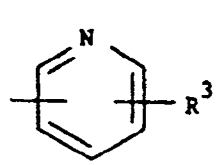

and $R^2$ is hydrogen or an acyl group of the formula $R^5CO$- where $R^5$ is hydrogen or $C_{1-4}$ alkyl, for use as a pharmaceutical.

4. The use of a compound according to claim 3, for the manufacture of a medicament for the treatment of an autoimmune disease or in the prevention of graft rejection.

5. A compound according to claim 1 in which $R^1$ is $C_{1-4}$ alkyl and $R^2$ is hydrogen or an acyl group of the formula $R^5CO$- where $R^5$ is hydrogen or $C_{1-4}$ alkyl, for use as a pharmaceutical.

6. The use of a compound according to claim 5 for the manufacture of a medicament for the treatment of autoimmune diseases or in the prevention of graft rejection.

7. A pharmaceutical composition comprising a compound as defined in claim 1, together with a pharmaceutically-acceptable diluent or carrier therefor.

8. A compound of the formula

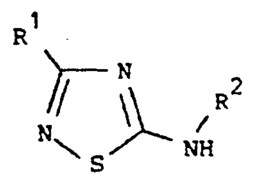

(I)

in which $R^1$ is hydrogen, $C_{1-4}$ alkyl, $C_{1-4}$ alkoxy-$C_{1-4}$ alkyl, hydroxy-$C_{1-4}$ alkyl, optionally substituted phenyl, optionally substituted phenyl-$C_{1-4}$ alkyl or a heterocycle selected from:

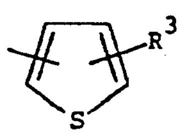
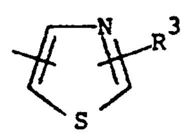
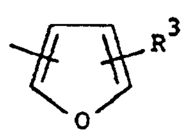
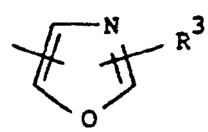
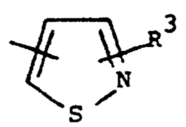
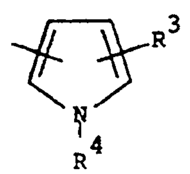
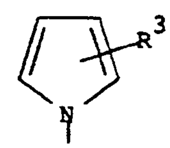
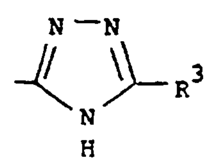
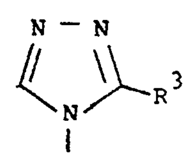
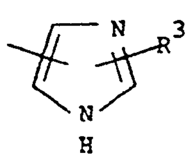
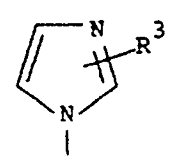
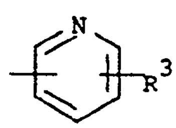
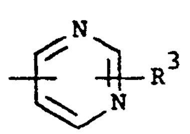
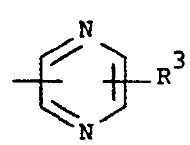
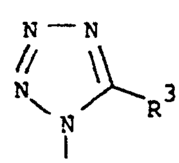
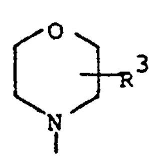
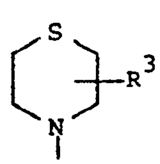

where $R^3$ is hydrogen, $C_{1-4}$ alkyl, $C_{1-4}$ alkoxy, nitro, halo, cyano, trifluoromethyl, carboxyl or $-CONH_2$, and $R^4$ is $C_{1-4}$ alkyl; and $R^2$ is $C_{1-4}$ alkyl, phenyl or thienyl substituted with a carboxyl or $C_{1-4}$ alkoxy-carbonyl group, an acyl group of the formula $R^5CO$- where $R^5$ is hydrogen, $C_{1-4}$ alkyl, $C_{1-4}$ alkoxy-$C_{1-4}$ alkyl, halo

$C_{1-4}$ alkyl, $C_{1-4}$ alkoxy, halo-$C_{1-4}$ alkoxy, phenyl, phenyl-$C_{1-4}$ alkyl, -NH$C_{1-4}$ alkyl, -NH phenyl or

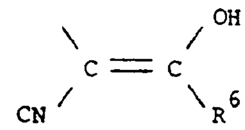

where $R^6$ is $C_{1-4}$ alkyl, or a group of the formula -CH=C(COC$_{1-4}$ alkoxy)$_2$; with the exception of compounds in which

(i) $R^1$ is hydrogen and $R^2$ is $CH_3CO$- or phenyl,
(ii) $R^1$ is methyl and $R^2$ is methyl or isopropyl,
(iii) $R^1$ is ethyl and $R^2$ is ethyl, isopropyl or $CH_3CO$-,
(iv) $R^1$ is propyl and $R^2$ is methyl, and
(v) $R^1$ is phenyl and $R^2$ is $C_{1-4}$ alkyl;

or a salt thereof.

**Claims for the following Contracting State: ES**

1. A process for preparing a pharmaceutical composition which comprises admixing a pharmaceutically-acceptable carrier or diluent with a compound of the formula

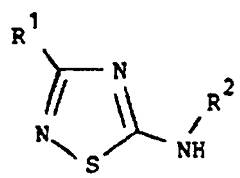

(I)

in which $R^1$ is hydrogen, $C_{1-4}$ alkyl, $C_{1-4}$ alkoxy-$C_{1-4}$ alkyl, hydroxy-$C_{1-4}$ alkyl, optionally substituted phenyl, optionally substituted phenyl-$C_{1-4}$ alkyl or a heterocycle selected from:

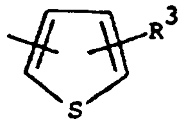
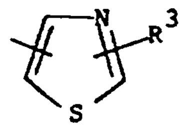
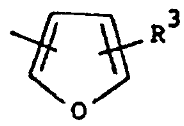
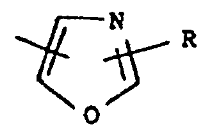
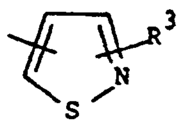
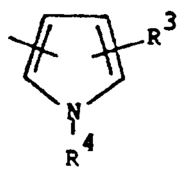
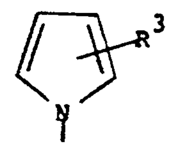
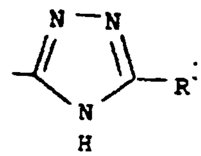
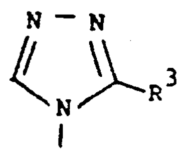
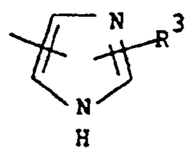
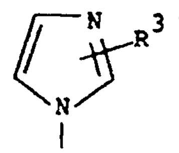
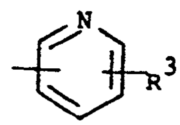
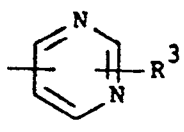
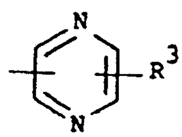
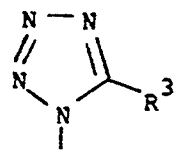
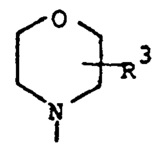
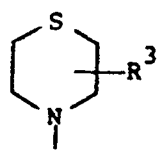

where $R^3$ is hydrogen, $C_{1-4}$ alkyl, $C_{1-4}$ alkoxy, nitro, halo, cyano, trifluoromethyl, carboxyl or $-CONH_2$, and $R^4$ is $C_{1-4}$ alkyl; and $R^2$ is hydrogen, $C_{1-4}$ alkyl, phenyl or thienyl substituted with a carboxyl or $C_{1-4}$ alkoxy-carbonyl group, an acyl group of the formula $R^5CO$- where $R^5$ is hydrogen, $C_{1-4}$ alkyl, $C_{1-4}$ alkoxy-$C_{1-4}$ alkyl, halo $C_{1-4}$ alkyl, $C_{1-4}$ alkoxy, halo-$C_{1-4}$ alkoxy, phenyl, phenyl-$C_{1-4}$ alkyl, $-NHC_{1-4}$ alkyl, -NH phenyl or

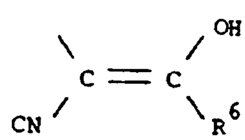

where $R^6$ is $C_{1-4}$ alkyl, or a group of the formula $-CH=C(COC_{1-4}\ alkoxy)_2$; or a salt thereof.

2. A process according to claim 1 for preparing a pharmaceutical composition by admixture of a compound of formula (I) in which $R^1$ is

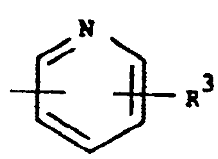

and $R^2$ is hydrogen or an acyl group of the formula $R^5CO$- where $R^5$ is hydrogen or $C_{1-4}$ alkyl.

3. A process according to claim 1 for preparing a pharmaceutical composition by admixture of a compound of formula (I) in which $R^1$ is $C_{1-4}$ alkyl and $R^2$ is hydrogen or an acyl group of the formula $R^5CO$- where $R^5$

is hydrogen or $C_{1-4}$ alkyl.

**4.** A process for producing a compound as defined in claim 1 with the exception of compounds in which

(i) $R^1$ is hydrogen and $R^2$ is $CH_3CO$- or phenyl,
(ii) $R^1$ is methyl and $R^2$ is methyl or isopropyl,
(iii) $R^1$ is ethyl and $R^2$ is ethyl, isopropyl or $CH_3CO$-,
(iv) $R^1$ is propyl and $R^2$ is methyl, and
(v) $R^1$ is phenyl and $R^2$ is $C_{1-4}$ alkyl;

or a salt thereof; which comprises

(a) aminating a compound of the formula

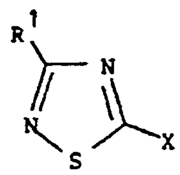

in which $R^1$ is hydrogen, $C_{1-4}$ alkyl, hydroxy-$C_{1-4}$ alkyl, $C_{1-4}$ alkoxy-$C_{1-4}$ alkyl, optionally substituted phenyl or a heterocycle, and X is a leaving group, with a compound of the formula $R^2NH_2$ where $R^2$ is hydrogen, $C_{1-4}$ alkyl or substituted phenyl or thienyl;

(b) reacting a compound of the formula

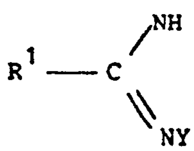

in which $R^1$ is hydrogen, $C_{1-4}$ alkyl, $C_{1-4}$ alkoxy-$C_{1-4}$ alkyl, optionally substituted phenyl or a heterocycle, and Y is halogen, with a thiocyanate, to give a compound of formula (I) in which $R^2$ is hydrogen; and

(c) acylating a compound of the formula

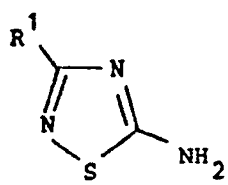

in which $R^1$ is hydrogen, $C_{1-4}$ alkyl, $C_{1-4}$ alkoxy-$C_{1-4}$ alkyl, optionally substituted phenyl or a heterocycle, with a reagent of the formula $R^2Z$ where Z is a leaving group, or $(R^2)_2O$, in which $R^2$ is an acyl group of the formula $R^5CO$-.

European Patent Office

# PARTIAL EUROPEAN SEARCH REPORT

which under Rule 45 of the European Patent Convention shall be considered, for the purposes of subsequent proceedings, as the European search report

Application number: EP 91 30 3089

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | CHEMISCHE BERICHTE, vol. 87, no. 1, 1954, pages 57-67, Weinheim, DE; J. GOERDELER et al.: "Über 1,2,4-Thiodiazole, I. Mitteil.): Darstellung und Eigenschaften der 5-Amino-1,2,4-thiodiazole"<br>* Page 63, 3rd paragraph from bottom; page 64, last paragraph; page 66, 2nd paragraph from bottom * | 8 | A 61 K 31/41<br>C 07 D 285/08<br>C 07 D 417/04 |
| X | CHEMISCHE BERICHTE, vol. 89, no. 6, 1956, pages 1534-1543, Weinheim, DE; J. GOERDELER et al.: "Darstellung und Eigenschaften des 1.2.4- und des 1.3.4-Thiodiazols"<br>* Page 1536, Formel III; page 1540 2nd paragraph from bottom *<br>./. | 8 | |

TECHNICAL FIELDS SEARCHED (Int. Cl.4)

C 07 D 285/00
C 07 D 417/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7
Claims searched incompletely: 8
Claims not searched:
Reason for the limitation of the search:

Given the extremely large number of already known compounds falling within the scope of claim 8, the Search Division considers that an exhaustive search or search report with respect to this claim would be economically justified (see Guidelines B III 2.1 and 3.7)

| Place of search | Date of completion of the search | Examiner |
|---|---|---|
| THE HAGUE | 16-07-1991 | ALLARD |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1. 03.82

European Patent Office

PARTIAL EUROPEAN SEARCH REPORT

Application number
EP 91 30 3089
-2-

| Category | Citation of document with indication, where appropriate, of relevant passages | Relevant to claim | CLASSIFICATION OF THE APPLICATION (Int. Cl.4) |
|---|---|---|---|
| X | BULLETIN DE LA SOCIETE CHIMIQUE DE FRANCE, no. 5, 1968, pages 2062-2066, Paris, FR; J.-L. DEROCQUE et al.: "Composés organiques sulfurés. XXII. -- Diaryl-2,5 trithia-1,6,6a$S^{IV}$ diaza-3,4 pentalènes" * Page 2065, table III, compounds 6-1,6-4B and 6-5B * -- | 8 | |
| X | CHEMICAL ABSTRACTS, vol. 68, no. 15, 8th April 1968, page 6663, abstract no. 69001x, Columbus, Ohio, US; & CZ-A- 120 541 (M. ZBIROVSKY et al.) 15-11-1966 * Whole abstract * -- | 8 | TECHNICAL FIELDS SEARCHED (Int Cl 4) |
| X | COLLECTION CZECHOSLOV. CHEM. COMMUN., vol. 36, no. 12, 1971, pages 4091-4098, Prag, CZ; M. ZBIROVSKY et al.: "Organische Herbizide VI. 3,5-Disubstituierte 1,2,4-Thiadiazole" * Page 4092, no. III; page 4093, no. XV; page 4094, nos. XXXII-XXXV * -- | 8 | |
| X | FR-A-2 261 278 (BAYER) * Pages 10,11 * -- | 8 | |
| X | DE-A-2 154 852 (MAKHTESHIM BEER-SHEVA CHEMICAL WORKS LTD) * Examples 13-15,19 * -- | 8 | |
| | ./. | | |

EPO Form 1505.3 06.78

European Patent Office

PARTIAL EUROPEAN SEARCH REPORT

Application number
EP 91 30 3089
-3-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | US-A-3 873 299 (BAYER)<br>* Whole document *<br>-- | 8 | |
| X | FR-A-1 598 962 (BAYER)<br>* Whole document *<br>-- | 8 | |
| X | DE-A-2 037 474 (ESSO RESEARCH)<br>* Whole document *<br>-- | 8 | TECHNICAL FIELDS SEARCHED (Int. Cl.4) |
| X | JOURNAL OF MEDICINAL CHEMISTRY, vol. 11, May 1968, pages 608-611, Washington, DC, US; E. FALCH et al.: "Substituted heteroaromatic anthranilic acids with antiinflammatory activity"<br>* Whole article, particularly page 610, table III, compound 24 *<br>-- | 1-8 | |
| P,X | CHEMICAL ABSTRACTS, vol. 114, no. 11, 18th March 1991, page 74, abstract no. 95162p, Columbus, Ohio, US; & JP-A-2 229 113 (H. TAGUCHI) 11-09-1990<br>* Whole abstract *<br>----- | 1-8 | |

EPO Form 1505.3 06.78